# EUROPEAN PATENT APPLICATION

(11) **EP 2 902 027 A1**
(43) Date of publication of application: **05.08.2015**
(21) Application number: 13840987.5
(22) Date of filing: 27.09.2013
(51) Int. Cl.: A61K 31/4748, A61K 31/704, A61P 35/00

(54) **DRUG COMPOSITION FOR TREATING TUMORS AND APPLICATION THEREOF**

(30) Priority: 28.09.2012 WO PCT/CN2012/082404
(71) Applicant: Hangzhou Bensheng Pharmaceutical Co., Ltd., Hangzhou, Zhejiang 310051 (CN)
(72) Inventor: XU, Rongzhen, Hangzhou Zhejiang 310012 (CN); RONG, Frank, Hangzhou Zhejiang 310012 (CN); XIE, Fuwen, Long Yan Fujian 364200 (CN)
(74) Representative: Cabinet Plasseraud
(86) International application number: PCT/CN2013/084547
(87) International publication number: WO 2014/048379

(57) **Abstract**

The present invention belongs to the field of medicine and pharmaceutical chemistry, specifically relates to novel antitumor pharmaceutical combination, and particularly relates to pharmaceutical combination of bisbenzylisoquinoline alkaloids (e.g. berbamine and tetrandrine) and anthracene compounds (e.g. aclarubicin A) and their use in treating tumors.

## Description

### Technical Field

The present invention belongs to the field of medicine and pharmaceutical chemistry, relates to novel antitumor pharmaceutical combination, and particularly relates to pharmaceutical combination of bisbenzylisoquinoline alkaloids and anthracene compounds and their use in treating tumors.

### Background of the Invention

Bisbenzylisoquinoline alkaloids are a class of very important natural products. They are derived from a wide range of medicinal materials, and can be separated, extracted, and characterized with mature processes. A plurality of these compounds with determined structures have been used as medicines for clinical applications, such as berbamine, tetrandrine, and fangchinoline.

Berbamine (BBM), also known as 6,6',7-trimethoxy-2,2'-dimethylberbaman-12-ol, is a bisbenzyl isoquinoline alkaloid extracted from Chinese herbal plants of berberis.

Berbamine has the effect of stimulating myeloid cell proliferation, improving the level of hematopoietic stem cell colony stimulating factor (GCSF), promoting proliferation of bone marrow hematopoietic stem cells and myeloid progenitor cells as well as their differentiation to granulocytes, and promoting proliferation of leukocytes (LIN Chuanrong, et al., The clinical observations on the treatment of chemotherapy-induced leukopenia with Shengbaijiao (berbamine), Prepared Chinese Medicine, 1994, 16 (7): 29).

Berbamine inhibits the proliferation of prostate cancer PC-3 cells by means of inducing cell apoptosis and affecting the cell cycles, and exhibits time- and concentration-dependencies (SUN Peng et al, Induction of apoptosis by berbamine in androgen-independent prostate cancer cell PC-3 in vitro, Chin J Exp Surg, August 2007, Vol 24, No. 8, pp 957).

Berbamine has significant anti-proliferation effect and specific apoptosis-inducing effect on K562 cells in vitro, and exhibits time-dependency. In a nude mouse bearing the tumor, berbamine also shows significant growth inhibiting effect to the K562 cells, and can particularly reduce the expression level of the tumor histocyte bcr/abl mRNA (WU Dong et al, Effects of Berbamine on K562 Cells and Its Mechanisms in vitro and in vivo, Journal of Experimental Hematology 2005, 13 (3): 373-378).

Berbamine has effect of inhibiting cytotoxic T lymphocytes, and significantly promotes mice natural killer cell activity in vitro, and can induce relative high level of interleukin II (IL-2) in vitro and in vivo and avoid the toxic and adverse effects induced by large doses of IL-2 for treatment of tumor. It is demonstrated experimentally that berbamine has a good protective effect for immune system in mice against radiation damage [LIU Xin, et al., The immune regulation action of berbamine on BALB/C mice, Journal of China Medical University, 1996, 25 (3): 229; LUO Chongnian, et al., The inhibition of berbamine on mice splenocytes cytotoxic T lymphocyte activity, Chinese Journal of Pharmacology and Toxicology, 1995, 9 (2): 159-160; GE Mingzhu, et al., The experimental study of immune protection action of berbamine on irradiated mice, Journal of Immunology, 1998, 14 (4): 238].

There are also research reports about the mechanisms of berbamine inducing the apoptosis of human leukemia Jurkat cells. Results show that berbamine can selectively inhibit the apoptosis of human leukemia Jurkat cells, arrest the cell cycle at S phase, and increase the protein expression of the cell caspase-3. With the active drug concentration increases from 0.5 µg/mL to 10 µg/mL, the cell viability decreases from 93.69% to 14.85%. Within this range of drug concentration, berbamine has no evident cytotoxic effect on the peripheral blood leucocyte of a normal human being (HUANG Zhiyu et al, Experimental Study of Berbamine Inducing Apoptosis in Human Leukemia Jurkat Cells, China Cancer, 2007, 16(9), 722).

Fangchinoline (FAN), also known as hanfangichin, is a bisbenzyl isoquinoline alkaloid extracted from Chinese herbal powder of fangji root. Fangchinoline is a natural calcium antagonist and has significant therapeutic effects on various diseases, such as broad-spectrum anti-inflammation, anti-hypertension, diabetes, liver fibrosis, and protection of brain cells. Research over recent years has discovered that it also has significant antitumor effects.

Studies have reported the inhibitory effect of fangchinoline on human colon cancer cell line LoVo. Tumor-bearing nude mice were administered with fangchinoline in the experiments continuously for 60 days, and the results showed that, as compared with the control group, the treated group exhibited significantly reduced and slow-growing tumor and lower microvessel density (WANG Zhenjun et al, Use of fangchinoline in angiogenesis inhibiting medicaments, [P] CN 1511528A. 2004-07-14).

Changdong Wang, et al. studied the inhibitory effect of fangchinoline on human prostate cancer cell PC3. Studies on cell cycle progress showed that the inhibitory effect of fangchinoline on PC3 cells was relevant to the increase of G1/S phase cells. In addition, fangchinoline had in vivo antitumor activity, reduced the tumor volume and promoted the apoptosis thereof, and inhibitory effect on the transplanted tumor PC3 in nude mice (Changdong Wang, et al. Fangchinoline induced G1/S arrest by modulating expression of p27, PCNA, and cyclin D in human prostate carcinoma cancer PC3 cells and tumor xenograft. Biosci. Biotechnol. Biochem. 2010, 74(3):488-493).

TANG Yalin et al studied the combination of G-quadruplex DNA and fangchinoline to increase the stability of G-quadruplex, thus weakening the ability of cell proliferation and promoting its apoptosis. In vitro anticancer cell proliferation experiments showed that the combination of G-quadruplex DNA and fangchinoline has better inhibitory effect on human leukemia cell line HL-60, human gastric carcinoma cell line BGC-823, human hepatocellular carcinoma cell line Bel-7402 and human nasopharyngeal carcinoma cell line KB (TANG Yalin et al, New use of bisbenzylisoquinoline alkaloid, [P] CN 101371839A. 2009-02-25).

Tetrandrine, or TTD or TE, with the chemical formula of 6,6',7,12-tetramethoxy -2,2'dimethylberbamine, is a bisbenzylisoquinoline alkaloid extracted from the root block of the Chinese herbal fangji.

Tetrandrine exhibits inhibition on the proliferation of cervical cancer HeLa cells. The studies use the MTT method to detect inhibition on the proliferation of the cervical cancer HeLa cell lines by tetrandrine in various concentrations and at different times. As is shown by the experiments, tetrandrine exhibits inhibition on proliferation of the cervical cancer HeLa cells, which exhibits time- and concentration-dependencies. (ZHU Kexiu et al., Qualitative and quantitative studies on tetrandrine-induced apoptosis of cervical cancer cells, Journal of Xi'an Jiaotong University (Medical Sciences Edition), 2010, 31 (1), 102).

Tetrandrine can inhibit the proliferation of hepatoma cells. After action of tetrandrine on the hepatoma cells, reactive oxygen species (ROS) are generated within 2 hours, and the production of ROS increases significantly with the increase of dose. It is suggested that tetrandrine may generate reactive oxygen species by interfering with the mitochondrial function, which causes the cell lipid peroxidation, damages the DNA molecules or regulates related genes of apoptosis, thereby inhibiting the proliferation of hepatoma cells. (Jing Xubin et al., Experimental studies on the tetrandrine-induced oxidative damage of hepatoma cells, Journal of Clinical Hepatology, 2002, 18 (6), 366).

On the other hand, anthracene compounds with anthraceno six-membered rings and having side chains and amino sugars, such as daunorubicin, idarubicin, daunomycin phenylhydrazone, doxorubicin, epirubicin, deoxydoxorubicin, quelamycin, THP adriamycin, fluoroethyl doxorubicin, aclarubicin (Acla), doxycycline, and the like, show a wide range of antitumor activity. They have been intensively studied in induction of apoptosis, immunoregulation and the mechanism of occurrence of drug resistance, and toxicity and side effects.

Aclarubicin A (ACM-A) is an antitumor antibiotics isolated from Galilee Streptomyces MA-144-M1 (ATCC31133). Research by Oki T et al showed that ACM-A had good antitumor effect. The leukemic cell lines L1210 and P388 were inoculated into mice, which were dosed for 10 consecutive days of 0.5-5mg/kg/day of ACM-A. As compared with those without antibiotics injection, the survival rate of injected mice increased by 150%. In addition, ACM-A also has significant effects on lymphoma 6C3HED, liver cancer, esophageal cancer, sarcoma-180, etc. (Oki T. US 3988315, 1976-10-26).

Aclarubicin A (ACM) belongs to the class of anthracene quinone antitumor antibiotics, which has much lower toxicity to the heart than that of adriamycin and daunomycin. ACM-A can significantly inhibit the growth of in vitro cultured mouse leukemia P₃₈₈ and human gastric cancer cell line SGC-7901. Cloning formation could be inhibited by 65.4% at a concentration of 0.01*µ*g/ml, wherein ED₅₀ is 0.0085 *µ*g/ml. ACM-A had significant therapeutic effects on leukemia mice, prolonging the lifetime of the mice by 143%, and half of the mice were treated. ACM-A also had strong inhibitory effect on Ehrlich ascites carcinoma and sarcoma-180. (Yang Jinlong et al., Domestic Aclarubicin A in vitro and in vivo antitumor effects, Acta Pharmaceutica Sinica, 1988, 23 (5): 321-326)

Aclarubicin A (ACM) has a growth inhibiting effect on a variety of animal tumors and human tumor transplantation in nude mice. The initial treatment of acute lymphoblastic leukemia (ANLL) achieves a complete remission rate (CR) of up to 70%; the ACM combined chemotherapy for treated ANLL achieves a CR of 23%-47%. This medicine is also effective for myelodysplastic syndrome, malignant lymphoma, breast cancer, and gastric cancer. (PAN Qichao, Pharmacology and clinic, Aclarubicin A China Pharmaceutical Journal, 1993, 2 (3): 25-28).

Aclarubicin A has obvious treatment effects on acute leukemia, lymphosarcoma, and the metastatic tumors of stomach, pancreas, intestine, ovary, lung and bladder, and also has good effects on liver cancer. (Eropob JIB, translated by WANG Tieliang, Aclarubicin A: new antitumor antibiotics, foreign medicine. Pharmacy section, 1983, 5: 297-298).

After oral and intravenous administration of ACM-A to rats, HPLC was applied to determine the level of ACM-A in tissues. It was found that the concentration in the tissues was higher than the blood drug level, reached maxium in lymph node and spleen, but kept a low concentration in lung, and reached minimum in the heart. The area under the curve for oral administration in the tissues was 2-7 times lower than that for intravenous injection. ACM-A was rapidly absorbed after oral administration, wherein the biological half-life of t_{1/2} =36.5h, and the bioavailability was 35%; after intravenous injection, the half-life t_{1/2}=16 - 21h. The concentrations of two metabolites increased steadily and rised above the concentration of the parent drug ACM-A after 12 - 18h. The cumulative bioavailability of ACM-A and the three metabolites was up to 89%. (Geodakyan S V, et al. Antibiotics and Medical Biotechnology, 1987, 32(10):768-72; Firsov A A, et al. Antibiotics and Medical Biotechnology, 1987, 32(10): 773-777).

On the other hand, drug combination has become the prevalent method of clinical treatment of AIDS. This method has many advantages, for example: (a) inhibiting HIV replication at multiple target sites and delaying the clinical progression of HIV infection; (b) reducing the probability of HIV drug resistance occurrence to the minimum; (c) significantly increasing the AIDS patients' quality of life and survival rate. The cocktail therapy proposed by Dr. David Ho is performed by the use of three or more antiviral drugs in combination for the treatment of AIDS. The application of this therapy can reduce the drug resistance from using one single drug, inhibit viral replication to the maximum, and cause partial or full recovery of the immune function of the damaged bodies, thus delaying the progression of disease, prolonging patients' lives, and improving the quality of life. More and more scientists believe that mixed drug therapy is the most effective treatment to AIDS by both inhibiting HIV reproduction and preventing the emergence of drug-resistant virus in vivo.

Because of the diversity of the patients and complexity of tumors, bisbenzylisoquinoline alkaloids and anthracycline compounds have shown their limitations while used alone (injection or oral), such as poor selectivity, low inhibition rate or low cure rate, high side effects or toxicity resulted from high doses, and the like. In order to solve the above problems, the present invention provides the combination of bisbenzylisoquinoline alkaloids and anthracycline compounds to improve the efficacy, selectivity and the cure rate in the treatment of tumors. At present, it has not yet been reported that the combination of bisbenzylisoquinoline alkaloids and anthracycline compounds is used for the treatment of tumor to improve the drug efficacy or cure rate. The objective of the present invention is to provide a "cocktail" or a composite medicine for treating tumors.

### Summary of the invention

One object of the present invention is to provide an antitumor pharmaceutical combination charaterzied by a bisbenzylisoquinoline alkaloid of Formula (I) and an anthracene compound of Formula (II) and pharmaceutically acceptable salts thereof, 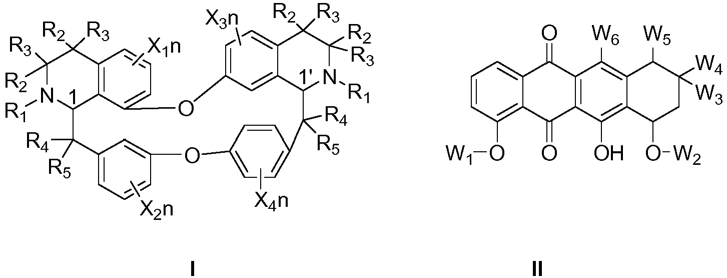 in Formula (I),
R₁ is H or linear or branched alkyl comprising 1 to 10 carbon atoms;
R₂ and R₃ each independently is H, substituted acyl, linear or branched alkyl, wherein the alkyl can be interrupted by O, N, S heteroatoms; or R₂ and R₃ together represents O or S;
R₄ and R₅ each independently is H, substituted acyl, linear or branched alkyl, wherein the alkyl can be interrupted by O, N, or S heteroatoms; or R₄ and R₅ together represents O or S;
X₁, X₂, X₃, and X₄ can be identical or different, each of which independently is halogen atoms, hydroxyl, or linear or branched alkyl comprising 1 to 10 carbon atoms, or alkoxy, or acyloxy, and n is an integer of from 0 to 4;
C(1) and C(1') are of a stereoisomer configuration selected from RR, SS, 1S1'R, 1R1'S;
in Formula (II),
W₁ is H, methyl, substituted or unsubstituted C₁-C₆ alkyl;
W₂ is H, glycosyl, or amino-substituted glycosyl;
W₃, W₄ each is H, hydroxyl, substituted or unsubstituted alkyl, or substituted carbonyl;
W₅ is H, substituted carbonyl or ester group;
W₆ is H, or hydroxyl;
wherein the aforementioned substituent(s) is(are) selected from the group consisting of halogen, amino, C₁-C₆ substituted amino, nitro, cyano, hydroxyl, C₁-C₆ alkoxy, mercapto, and C₁-C₆ alkylthio; wherein the substituent(s) can be one or more for each occurrence, identical or different, each substituting an H atom.

The bisbenzylisoquinoline alkaloid of Formula (I) can be selected from the following:

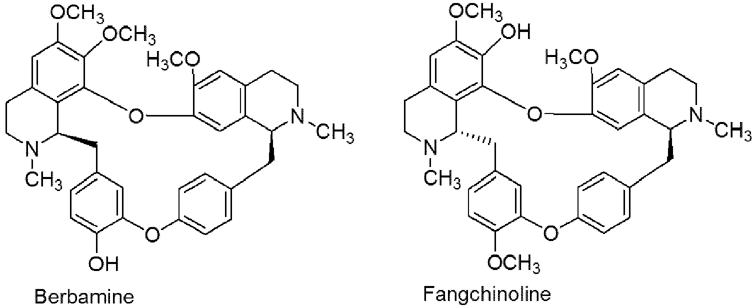

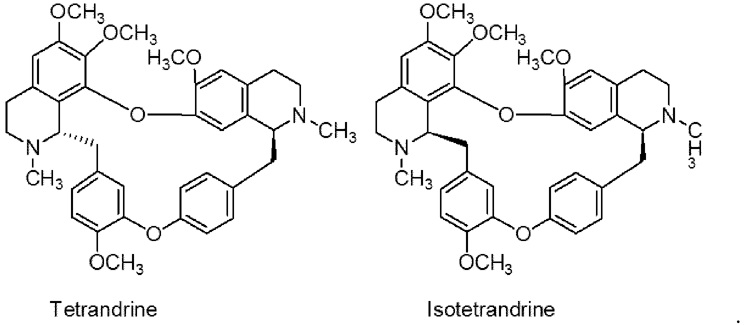

The anthracene compound of Formula (II) can be selected from the following:

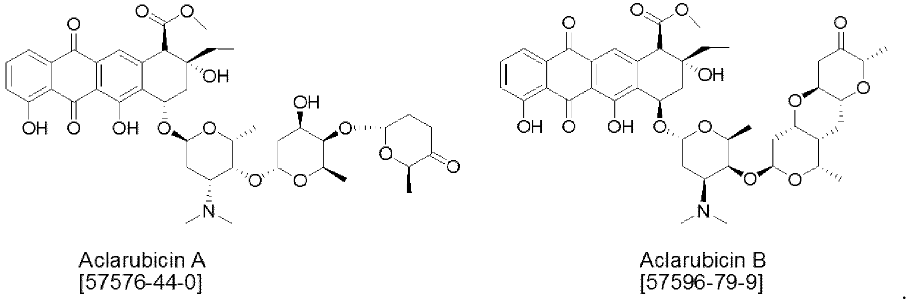

A second object of the present invention is to provide a suitable combination of the bisbenzylisoquinoline alkaloid of Formula (I) and the anthracene compound of Formula (II) as an antitumor medicament. The synergistic effect between the two, particularly the pharmaceutical combination of tetrandrine and aclarubicin, achieves better therapeutic effects.

A third object of the present invention is to provide the use, particularly the antitumor clinical use, of the pharmaceutical combination of the present invention. Correspondingly, the present invention provides a method for treating a subject suffering from tumor, comprising administrating to the subject in need thereof an effective amount of the pharmaceutical combination of the present invention, wherein the tumor is particularly selected from leukemia, multiple myeloma, lymphoma, liver cancer, gastric cancer, breast cancer, cholangiocellular carcinoma, pancreatic cancer, lung cancer, colorectal carcinoma, osteosarcoma, melanoma, human cervical cancer, glioma, nasopharyngeal carcinoma, laryngeal carcinoma, esophageal cancer, middle ear tumor, and prostate cancer.

### Brief description of the drawings

Figure 1A shows the dynamic curve of TTD's effects on the body weight of nude mice.
Figure 1B shows the dynamic curve of TTD's effects on the A549 transplated tumor volume of nude mice.
Figure 1C shows TTD's effects on the A549 transplated tumor size of nude mice.
Figure 1D shows TTD50's effects on the A549 transplated tumor weight of nude mice.
Figure 1E shows TTD's inhibitory effects on the A549 transplated tumor of nude mice.
Figure 2A shows the dynamic curve of TTD's effects on the Hep G2 transplated tumor volume of nude mice.
Figure 2B shows TTD's effects on the Hep G2 transplated tumor size of nude mice.
Figure 2C shows TTD50's effects on the Hep G2 transplated tumor weight of nude mice.
Figure 2D shows TTD's inhibitory effects on the Hep G2 transplated tumor of nude mice.
Figure 3A shows the dynamic curve of Acla A's effects on the body weight of nude mice.
Figure 3B shows the dynamic curve of Acla A's effects on the A549 transplated tumor volume of nude mice.
Figure 3C shows Acla A's effects on the A549 transplated tumor size of nude mice.
Figure 3D shows Acla A's effects on the A549 transplated tumor weight of nude mice.
Figure 3E shows Acla A's inhibitory effects on the A549 transplated tumor of nude mice.
Figure 4A shows the dynamic curve of the effects by the combination use of TTD with Acla A on the body weight of nude mice.
Figure 4B shows the dynamic curve of the effects by the combination use of TTD with Acla A on the A549 transplated tumor volume of nude mice.
Figure 4C shows the effects by the combination use of TTD with Acla A on the A549 transplated tumor size of nude mice.
Figure 4D shows the effects by the combination use of TTD with Acla A on the A549 transplated tumor weight of nude mice.
Figure 4E shows the inhibitory effects by the combination use of TTD with Acla A on the A549 transplated tumor of nude mice.
Figure 5A shows the dynamic curve of the effects by the combination use of TTD with Acla A on the Hep G2 transplated tumor volume of nude mice.
Figure 5B shows the effects by the combination use of TTD with Acla A on the Hep G2 transplated tumor size of nude mice.
Figure 5C shows the effects by the combination use of TTD with Acla A on the Hep G2 transplated tumor weight of nude mice.
Figure 5D shows the inhibitory effects by the combination use of TTD with Acla A on the Hep G2 transplated tumor of nude mice.

### Detailed description of the invention

The present invention provides an antitumor pharmaceutical combination, which is charaterzied by comprising a bisbenzylisoquinoline alkaloid of Formula (I) and an anthracene compound of Formula (II), 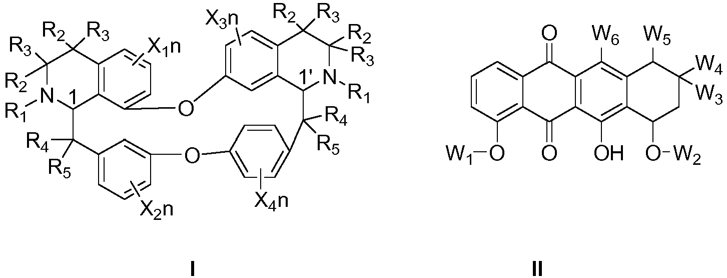 in Formula (I),
R₁ is H or linear or branched alkyl comprising 1 to 10 carbon atoms;
R₂ and R₃ each independently is H, substituted acyl, linear or branched alkyl, wherein the alkyl can be interrupted by O, N, S heteroatoms; or R₂ and R₃ together represents O or S;
R₄ and R₅ each independently is H, substituted acyl, linear or branched alkyl, wherein the alkyl can be interrupted by O, N, or S heteroatoms; or R₄ and R₅ together represents O or S;
X₁, X₂, X₃, and X₄ can be identical or different, each of which independently is halogen atoms, hydroxyl, or linear or branched alkyl comprising 1 to 10 carbon atoms, or alkoxy, or acyloxy, and n is an integer of from 0 to 4;
C(1) and C(1') are of a stereoisomer configuration selected from RR, SS, 1S1'R, 1R1'S; and
in Formula (II),
W₁ is H, methyl, substituted or unsubstituted C₁-C₆ alkyl;
W₂ is H, glycosyl, or amino-substituted glycosyl;
W₃, W₄ each is H, hydroxyl, substituted or unsubstituted alkyl, or substituted carbonyl;
W₅ is H, substituted carbonyl or ester group;
W₆ is H, or hydroxyl;
wherein the aforementioned substituent is selected from the group consisting of halogen, amino, C₁-C₆ substituted amino, nitro, cyano, hydroxyl, C₁-C₆ alkoxy, mercapto, and C₁-C₆ alkylthio; wherein the substituent(s) can be one or more for each occurrence, identical or different, each substituting an H atom.

The bisbenzylisoquinoline alkaloid of Formula (I) can be selected from the following:

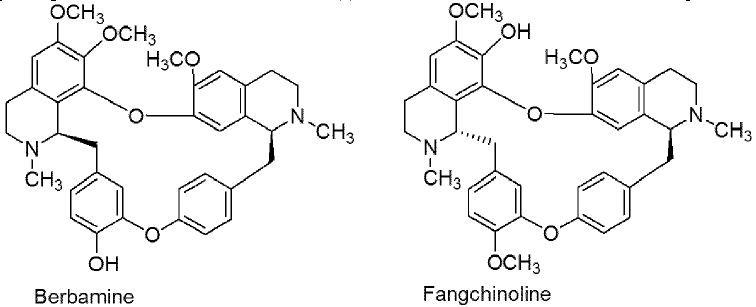

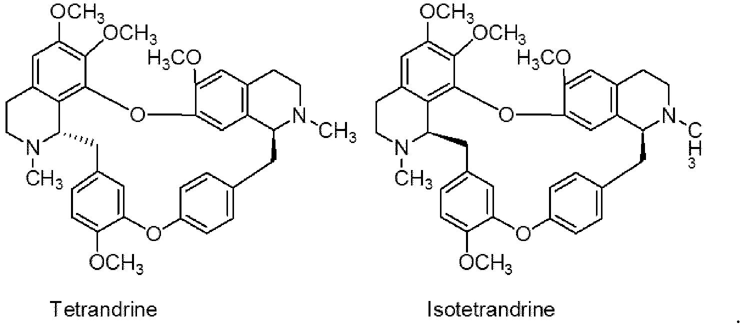

The anthracene compound of Formula (II) can be selected from the following:

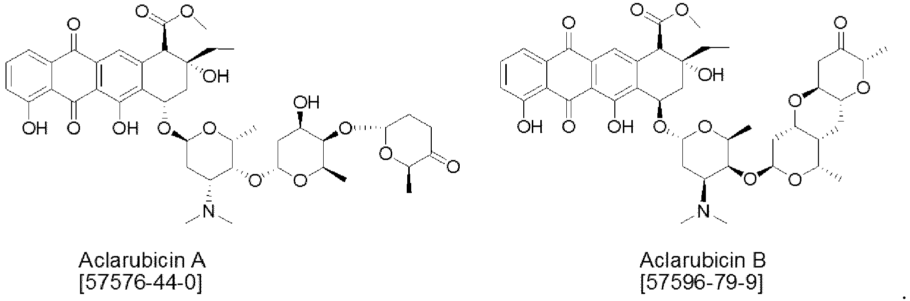

The bisbenzylisoquinoline alkaloid of Formula (I) can be prepared and characterized in structure and activity according to the methods described in, such as, CN1903856A, US 6617335 B1, or CN101371839A and characterized by the structure and activity thereof. The anthracene compound of Formula (II) can be prepared and characterized in structure and activity according to the methods described in, such as, US4375511A and Acta Pharmaceutica Sinica 1988,23(5):321-326.

A suitable combination of the bisbenzylisoquinoline alkaloid of Formula (I) and the anthracene compound of Formula (II) of the present invention is used as an antitumor medicament. The synergistic effect between the two, particularly the pharmaceutical combination of tetrandrine and aclarubicin, achieves better therapeutic effects.

The bisbenzylisoquinoline alkaloid of Formula (I) can be present in the pharmaceutical combination in the form of its corresponding salt formed with a pharmaceutically acceptable inorganic or organic acid.

The term "aromatic hydrocarbyl" refers to an aryl without a heteroatom, including aryl, arylalkyl, and alkylaryl.

The term "aromatic heterocycyl" refers to an aromatic hydrocarbyl with a heteroatom, including heterocyclic aryl, heterocyclic arylalkyl, and alkylheterocyclic aryl. The heteroatom refers to N, O, and S. An arylheterocyclic radical can comprise one or more heteroatoms.

The term "halogen", "halo" or "hal" means fluorine, chlorine, bromine or iodine.

The term "C₁-C₆ substituted amino" refers to -N- C₁-C₆ alkyl and -N-C₃-C₆cycloalkyl.

The term "C₁-C₆ alkoxy" refers to -O- C₁-C₆ alkyl and -O-C₃-C₆cycloalkyl.

The term "C₁-C₆ alkylthio" refers to -S- C₁-C₆ alkyl and -S-C₃-C₆cycloalkyl.

The term "a pharmaceutically acceptable salt" includes, but not limited to, tosylate, methanesulfonate, malate, acetate, citrate, malonate, tartrate, succinate, benzoate, ascorbate, α-ketoglutarate, and α-glycerophosphate. Suitable inorganic salts may also be formed, including but not limited to, hydrochlorate, sulfate, nitrate, bicarbonate and carbonate, phosphate, hydrobromate, hydriodate salts and the like.

The terms "treatment," "treating," "treat," and the like used herein refer generally to obtaining a desired pharmacological and/or physiological effect. The effect may be prophylactic in terms of completely or partially preventing a disease or symptoms thereof and/or may be therapeutic in terms of partial or complete stabilization or cure of a disease and/or adverse effects caused by the disease. "Treatment" as used herein covers any treatment of a disease in a subject, including: (a) preventing the disease or symptoms from occurring in a subject who is predisposed to the disease or symptoms but has not yet been diagnosed as having it; (b) inhibiting the symptoms of a disease, i.e., arresting its development; or (c) relieving the symptoms of a disease, i.e., causing regression of the disease or symptoms.

The present invention also provides a suitable combination of the bisbenzylisoquinoline alkaloid of Formula (I) and the anthracene compound of Formula (II) as an antitumor medicament. The synergistic effect between these two, particularly the pharmaceutical combination of tetrandrine and aclarubicin, achieves better therapeutic effects.

The present invention relates to the combination of two or more of the bisbenzylisoquinoline alkaloid of Formula (I) and the anthracene compound of Formula (II) as an antitumor medicament, which has a constitution and weight percentage as follows:
(1) tetrandrine comprises 0.1-80%, preferably 1-50%, of the active components;
(2) aclarubicin comprises 0.1-80%, preferably 1-50%, of the active components.

The present invention provides pharmaceutical combination that comprises at least two compounds as set forth above and optionally a pharmaceutically acceptable excipient.

The methods for preparing various pharmaceutical compositions having a certain amount of active components are known or will be apparent to those skilled in the art in light of this disclosure. As described in REMINGTON'S PHARMACEUTICAL SCIENCES, Martin, E.W., ed., Mack Publishing Company, 19th ed. (1995), the methods for preparing such pharmaceutical compositions include incorporation of other suitable pharmaceutical excipients, carriers, diluents, etc.

The pharmaceutical preparations of the present invention are produced by known methods, including routine mixing, dissolving, or lyophilizing processes.

The pharmaceutical combination of the present invention may be formulated into a pharmaceutical composition and administered to a patient in a route suitable for the selected administration manner, e.g., orally or parenterally (by an intravenous, intramuscular, topical or subcutaneous route).

Thus, the pharmaceutical combination of the present invention may be systemically administered, e.g., orally, in conjugation with a pharmaceutically acceptable carrier such as an inert diluent or an assimilable edible carrier. They may be enclosed in hard or soft gelatin capsules, or may be compressed into tablets. For oral therapeutic administration, the active compound may be combined with one or more excipients and may be taken in a form of ingestible tablet, buccal tablet, troche, capsule, elixir, suspension, syrup, wafer, and the like. Such a composition and preparation should contain at least 0.1% of the active compound. This proportion of the compositions and preparations may, of course, vary and may conveniently be from about 1% to about 99% by the weight of a given unit dosage form. The active compound is present in such a therapeutically useful composition in an amount such that an effective dosage level is achieved.

A tablet, troche, pill, capsule and the like may also comprises a binder such as gum tragacanth, acacia, corn starch or gelatin; an excipient such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, wintergreen oil, or cherry flavor. When being a capsule as the unit dosage form, it may comprise, in addition to the above material types, a liquid vehicle such as a vegetable oil or polyethylene glycol. Various other materials may be present as coatings or otherwise modify the physical form of the solid unit dosage form. For instance, a tablet, pill, or capsule may be coated with gelatin, wax, shellac or sugar, etc. A syrup or elixir may contain an active compound, a sweetening agent such as sucrose or fructose, a preservative such as methylparaben or propylparaben, a dye and a flavoring agent (such as cherry or orange flavor). Of course, any materials used in preparing any unit dosage form should be pharmaceutically acceptable and substantially non-toxic in the amounts employed. In addition, the active compound may be incorporated into a sustained-release preparation and device.

The active compound may also be administered by infusion or injection intravenously or intraperitoneally. An aqueous solution of the active compound or its salt may be prepared, optionally mixed with a nontoxic surfactant. A dispersion can also be prepared in glycerol, liquid polyethylene glycol, triacetin, and a mixture thereof and in an oil. Under ordinary storage and use conditions, these preparations contain a preservative to prevent the growth of microorganisms.

The pharmaceutical dosage forms suitable for injection or infusion can include a sterile aqueous solution or dispersion or a sterile powder comprising the active ingredient (optionally encapsulated in liposomes) which are adapted for an extemporaneous preparation of a sterile injectable or infusible solution or dispersion. In all cases, the final dosage form must be sterile, liquid and stable under the manufacture and storage conditions. The liquid carrier or vehicle may be a solvent or a liquid dispersion medium comprising, for example, water, ethanol, a polyol (for example, glycerol, propylene glycol, liquid polyethylene glycol, and the like), a vegetable oil, a nontoxic glyceryl ester, and a suitable mixture thereof. The proper fluidity can be maintained, for example, by formation of liposomes, by maintenance of the required particle size in the case of dispersion or by the use of a surfactant. The prevention of microorganism action can be achieved by various antibacterial and antifungal agents, such as parabens, chlorobutanol, phenol, sorbic acid, thimerosal, and the like. In many cases, it will be preferable to include an isotonic agent, such as a sugar, a buffer agent or sodium chloride. Prolonged absorption of an injectable composition can be obtained by the use of a composition of the agents for delaying absorption, for example, aluminum monostearate and gelatin.

A sterile injectable solution is prepared by combining the active compound in a required amount in a suitable solvent with various additional components as listed above as required, followed by filter sterilization. In the case of sterile powder for preparation of a sterile injectable solution, the preferred preparation process is the vacuum drying and lyophilization techniques, which yield a powder of the active ingredient plus any additional desired ingredient present in the previously sterile-filtered solution.

Useful solid carriers include finely divided solids such as talc, clay, microcrystalline cellulose, silica, alumina and the like. Useful liquid carriers include water, ethanol or ethylene glycol or a water-ethanol/ ethylene glycol mixture, in which the compound of the present invention can be dissolved or dispersed at an effective level optionally with the aid of a non-toxic surfactant. An adjuvant (such as a flavour) and an additional antimicrobial agent can be added to optimize the properties for a given application.

A thickening agent (such as a synthetic polymer, a fatty acid, a fatty acid salt and ester, a fatty alcohol, a modified cellulose or a modified mineral) can also be used with a liquid carrier to form a spreadable paste, gel, ointment, soap and the like for application directly to the skin of a user.

The treatment required amount of the compound or an active salt or derivative thereof will vary depending not only on the selected particular salt but also on the administration route, the nature of the condition to be treated and the age and condition of the patient, and will be ultimately determined at the discretion of the attendant physician or clinician.

The above formulations can be present in a unit dosage form which is a physically discrete unit containing a unit dosage suitably administrating to a human or other mammalians. The unit dosage form may be a capsule or a tablet, or a plurality of capsules or tablets. Depending upon the intended particular treatment, the amount of the active ingredient in a unit dosage form can be varied or adjusted in the range of about 0.1 mg to about 1,000 mg or more.

If the pharmaceutical composition of the present invention is made into a formulation and administered intestinally or non-intestinally, then each formulation can have the active components of the following contents, calculated based on basic unit of measurement:
(a) bisbenzylisoquinoline alkaloid: 0.1 - 150 mg;
(b) anthracene compound: 0.1 - 150 mg.

For example,
(a) tetrandrine: 0.1 - 150 mg;
(b) aclarubicin: 0.1 - 150 mg.

In the formulation of the pharmaceutical composition of the present invention, the content of each main component can be, but not limited to, the following:
bisbenzylisoquinoline alkaloid: 1-50% ;
anthracene compound: 1-50% ;
starch: 1-60% ;
dextrin: 0-20% ;
microcrystalline cellulose: 0-25% ;
sodium carboxymethyl starch: 0-5% ;
polyethylene glycol: 0-5% ;
hydroxypropyl methyl cellulose: 0-5% ;
magnesium stearate: 0-5%.

The present invention also provides the use, particularly the antitumor use, of the pharmaceutical composition of the present invention. Correspondingly, the present invention provides a method for treating a subject suffering from tumor, comprising administrating to the patient in need thereof an effective amount of the pharmaceutical combination of the present invention. The pharmaceutical combination of the bisbenzylisoquinoline alkaloid of Formula (I) and the anthracene compound of Formula (II) of the present invention can be used to treat, for example, leukemia, multiple myeloma, lymphoma, liver cancer, gastric cancer, breast cancer, cholangiocellular carcinoma, pancreatic cancer, lung cancer, colorectal carcinoma, osteosarcoma, melanoma, human cervical cancer, glioma, nasopharyngeal carcinoma, laryngeal carcinoma, esophageal cancer, middle ear tumor, prostate cancer, among other tumors.

In the following examples, the present invention will be explained in more detailed. However, it should be understood that the following examples are intended to illustrate the present invention but not to limit the scope of the present invention in any way.

The raw chemicals used in the following examples are commercially available.

### Example

### Part I: Inhibitory effects by the combination use of TTD with Acla A on tumor cells in vitro

### Example 1: Pharmaceutical combination of TTD and Acla A for the inhibition of leukemia cells

(1) Experimental materials
(2) Leukemia cell lines: K562/adr (drug-resistant chronic myeloid leukemia (CML), Kasumi-1 (acute myeloid leukemia M2 type, AML-M2), Jurkat (acute lymphoblastic leukemia, ALL), all of which are donated by Cancer Research Institute of Zhejiang University, China.
(3) Reagents:
TTD (tetrandrine, Jiangxi Jinfurong Pharmaceutical Co., Ltd.);
Acla A (aclarubicin A, Shenzhen Main Luck Pharmaceuticals Inc.)

(4) Main apparatuses:
Cell incubator (Thermo Scientific 3111)
Biosafety cabinet (Heal Force, Hfsafe-1200A2)
Microplate reader (Bio-rad, Imark)

(5) Experimental method
a) Determine IC₅₀ value after 72 hours of the separated comopunds on leukemia cells. Well-growing leukemia cells were obtained and inoculated into wells of a 96-well cell culture plate at 5000 cells/well. Individual compounds of various concentrations were added, mixed uniformly, and incubated (5% CO₂) at 37°C for 72 hours. Then the cell viability was determined by the MTT method. In this experiment, the cell viability in control group (not treated with any compound) was set as 100%, and the half maximal inhibitory concentrations of TTD and Acla A for the leukemia cell growth after 72 hours (IC₅₀ value of 72 hours, µg/mL) were calculated.
b) Determine the inhibitory effects of TTD in combination with Acla A on leukemia cells in vitro. The pretreatment of the cells was the same as in a). The control group was not treated with the compound while administered. The two single-drug groups were added with respective active component to the concentration under which the IC₅₀ of two compounds in a) achieves. As to the combination group, two compounds were added together into the same group of wells, with the final concentration being the concentration under which the IC₅₀ of the single drugs achieves. After 72 hours of incubation, the MTT method was used for the determination of cell viability. The cell viability of the control group was set to 100% in this experiment, and the TTD, Acla A single drug and combined effects on cell viability (%) were calculated.

(6) The experimental results
The experimental results are shown in Table 1.
As is seen from Table 1, for the leukemia cell lines tested, the IC₅₀ dose of TTD in combination with Acla A resulted in significant lower cell viability than either TTD or Acla A used alone. In addition, in the case of k562/ADR, after the combined effects of TTD and Acla A at IC₅₀ dosage, the cell viability is reduced by almost 30% as compared with single drug.

**Table 1: Determination of the inhibitory concentrations of TTD in combination with Acla A on leukemia cells**

| | TTD | | Acla A | | TTD+Acla A | |
|---|---|---|---|---|---|---|
| Cell lines | concentration (µg/mL) | CV(%) | concentration (µg/mL) | CV(%) | concentration (µg/mL) | CV(%) |
| k562/ADR | 0.6 | 48.032 | 0.09 | 49.053 | 0.6+0.09 | 21.17 |
| Jurkat | 1.69 | 52.288 | 0.029 | 57.967 | 1.69+0.029 | 42.84 |
| kasumi-1 | 2 | 51.652 | 0.04 | 52.486 | 2+0.04 | 36.78 |

### Example 2: Pharmaceutical combination of TTD and Acla A for the inhibition of human lymphoma and multiple myeloma cells

(1) Experimental materials
Multiple myeloma cell lines: RPMI8226 (multiple myeloma), purchased from Fuxiang Bio-tech Co. Ltd., Shanghai, China; Raji (human Burkitt lymphoma cells), purchased from China Center For Type Culture Collection.
(2) Reagents:
Same as in Example 1.

(3) Main apparatuses:
Same as in Example 1.

(4) Experimental method
a) Determine IC₅₀ value after 72 hours of separate compounds on the above cells. Well-growing cells of above were obtained and inoculated into wells of a 96-well cell culture plate at 5000 cells/well. Separate compounds of various concentrations were added, mixed uniformly, and incubated (5% CO₂) at 37°C for 72 hours. Then the cell viability was determined by the MTT method. In this experiment, the cell viability in control group (not treated with any compound) was set as 100%, and the half maximal inhibitory concentrations of TTD and Acla A for the above cell growth at 72 hours (IC₅₀ value of 72 hours, µg/mL) were calculated.
b) Determine the inhibitory effects of TTD in combination with Acla A on the above cells. The pretreatment of the cells was the same as in a). The control group was not treated with the compound while administered. The two single-drug groups were added with respective active component to the concentration under which the IC₅₀ of two compounds in a) achieves. As to the combination group, two compounds were added together into the same group of wells, with the final concentrations being the concentrations to which the IC₅₀ values of the two single drugs correspond. After 72 hours of incubation, the MTT method was used for the determination of cell viability. The cell viability of the control group was set to 100% in this experiment, and the TTD, Acla A single drug and combined effects on cell viability (%) were calculated.

(5) The experimental results
The experimental results are shown in Table 2.
As is seen from Table 2, for the lymphoma and myeloma cell lines tested, the IC₅₀ dose of TTD in combination with Acla A resulted in significant lower cell viability than either TTD or Acla A used alone. In addition, in the case of Raji and RPMI 8226, after the combined effects of TTD and Acla A at IC₅₀ dosage, the cell viability was reduced by almost 20% as compared with single drug.

**Table 2**

| | TTD | | Acla A | | TTD+Acla A | |
|---|---|---|---|---|---|---|
| Cell lines | concentration (µg/mL) | CV(%) | concentration (µg/mL) | CV(%) | concentration (µg/mL) | CV(%) |
| Raji | 1 | 46.87 | 0.03 | 42.569 | 1+0.03 | 34.62 |
| RPMI 8226 | 0.3 | 53.31 | 0.035 | 42.056 | 0.3+0.035 | 34.89 |

### Example 3: Pharmaceutical combination of TTD and Acla A for the inhibition of human solid tumor cells

(1) Experimental materials
Human solid tumor cell lines: Hep-2 (laryngeal carcinoma), A549 (human lung cancer), CaES-17 (esophageal cancer cell), PC-3 (prostate cancer), CNE (nasopharyngeal carcinoma cell), SPC-A-1(human lung adenocarcinoma cell), and SK-OV-3 (ovarian cancer cell), all of which are purchased from China Center For Type Culture Collection; RKO (human colon adenocarcinoma cell), MGC 803 (human gastric cancer cell), MG63 (osteosarcoma), and U87 MG (malignant glioma cell), all of which are purchased from Fuxiang Bio-tech Co. Ltd., Shanghai, China; PANC-1 (pancreatic cancer), Huh7 (human liver cancer cell), Hep G2(human hepatoma carcinoma cell), Becap37 (human breast cancer cell), and Hela (human cervical cancer cell), all of which are donated by Cancer Research Institute of Zhejiang University, China.
(2) Reagents:
Same as in Example 1.

(3) Main apparatuses:
Same as in Example 1.

(4) Experimental method
a) Determine IC50 value after 72 hours of separate compound on the human solid tumor cells. Well-growing cells of above were obtained and inoculated into wells of a 96-well cell culture plate at 5000 cells/well. Individual compounds of various concentrations were added, mixed uniformly, and incubated (5% CO2) at 37oC for 72 hours. Then the cell viability was determined by the MTT method. In this experiment, the cell viability in control group (not treated with any compound) was set as 100%, and the half maximal inhibitory concentrations of TTD and Acla A for the human solid tumor cell growth at 72 hours (IC50 value of 72 hours, µg/mL) were calculated.
b) Determine the inhibitory effects of TTD in combination with Acla A on the human solid tumor cells. The pretreatment of the cells was the same as in a). The control group was not treated with the compound while administered. The two single-drug groups were added with respective active component to the concentration under which the IC50 of two compounds in a) achieve. As to the combination group, two compounds were added together into the same group of wells, with the final concentration being the concentration to which the IC50 of the single drugs correspond. After 72 hours of incubation, the MTT method was used for the determination of cell viability. The cell viability of the control group was set to 100% in this experiment, and the TTD, Acla A single drug and combined effects on cell viability (%) were calculated.

(5) The experimental results
The experimental results are shown in Table 3. As is seen from Table 3, for the 16 solid tumor cell lines tested, the IC₅₀ dose of TTD in combination with Acla A resulted in significant lower cell viability than either TTD or Acla A used alone. In addition, in the case of PC-3, after the combined effects of TTD and Acla A at IC₅₀ dosage, the cell viability was reduced by almost 40% as compared with single drug. In the case of Becap-37 and Huh-7, after the combined effects of TTD and Acla A at IC₅₀ dosage, the cell viability was reduced by almost 30% as compared with single drug.

**Table 3**

| | TTD | | Acla A | | TTD+Acla A | |
|---|---|---|---|---|---|---|
| Cell lines | concentration (µg/mL) | CV(%) | concentration (µg/mL) | CV(%) | concentration (µg/mL) | CV(%) |
| A549 | 8.34 | 47.592 | 0.08 | 50.37 | 8.34+0.08 | 38.03 |
| SPC-A-1 | 3.5 | 52.511 | 0.03125 | 50.584 | 3.5+0.03125 | 33.88 |
| Becap-37 | 3 | 56.106 | 0.5 | 44.766 | 3+0.5 | 21.05 |
| CaES-17 | 3.59 | 47.089 | 0.24 | 45.402 | 3.59+0.24 | 35.09 |
| CNE | 3 | 51.923 | 0.15 | 48.925 | 3+0.15 | 38.75 |
| Hela | 1.25 | 46.983 | 0.095 | 52.671 | 1.25+0.095 | 29.45 |
| Hep2 | 2.59 | 43.798 | 0.25 | 50.97 | 2.59+0.25 | 32.77 |
| Hep G2 | 1.5 | 52.499 | 0.25 | 51.598 | 1.5+0.25 | 34.62 |
| Huh7 | 3 | 53.984 | 0.05 | 44.457 | 3+0.05 | 18.02 |
| MG63 | 2 | 40.805 | 0.1 | 46.578 | 2+0.1 | 41.59 |
| MGC-803 | 3 | 50.857 | 0.1 | 42.243 | 3+0.1 | 33.67 |
| Panc-1 | 4.67 | 52.288 | 0.25 | 57.967 | 4.67+0.25 | 42.84 |
| PC-3 | 4.5 | 46.983 | 0.8 | 46.139 | 4.5+0.8 | 6.416 |
| RKO | 1.5 | 45.266 | 0.04 | 49.628 | 1.5+0.04 | 34.38 |
| SK-OV-3 | 5 | 55.869 | 0.25 | 53.443 | 5+0.25 | 44.17 |
| U87-MG | 2.37 | 47.701 | 0.21 | 54.068 | 2.37+0.21 | 46.82 |

### Part II: Inhibitory effects of TTD in combination with Acla A on tumor in vivo

### Example 4: TTD for the inhibition of lung cancer and liver cancer transplanted tumor in nude mice

(1) Experimental materials
Lung cancer cell lines: A549 (human non-small cell lung cancer cell line);
Liver cancer cell lines: Hep G2 (human hepatoma carcinoma cell);
Animals: BALB/c-nu nude mice (immunodeficient mice), 8 weeks, female, purchased from Shanghai Laboratory Animal Center of Chinese Academy of Sciences, China.

(2) Reagents:
Jef (Jefitinib, LC laboratories)
TTD (tetrandrine, Jiangxi Jinfurong Pharmaceutical Co., Ltd.);

(3) Main apparatuses:
Cell incubator (Thermo Scientific 3111)
Laminar flow rack (Suhang Brand clean animal breeding cabinet, Type DJ-2).

(4) Experimental method
Under sterile conditions, the above tumor cells were collected in the logarithmic growth phase. A549 was injected by subcutaneous injection in an amount of 1×10⁷/0.2 mL/nude mice (cell viability>90%) into the right subaxillary of the nude mice, and Hep G2 was injected by subcutaneous injection in an amount of 1×10⁷/0.2 mL/nude mice (cell viability>90%) into the left subaxillary of the nude mice thus establishing transplated tumor models of lung cancer and liver cancer respectively in nude mice. The administration was carried out from the second day after the inoculation. The experimental group was intragastrically administered at the experimentally designed amount, the negative control group is intragastrically administered with sterile water, and the positive control group was intragastrically administered with Jef. Each mouse was intragastrically administered in 0.4 ml each time and 3 times a day, at 8:00, 14:00 and 20:00. The administrations were successive for 10 days. The day before administration was deemed as Day 0 and the body weight and tumor size of the mice were determined every 5 days to produce a dynamic plot on body weight and tumor growth. On Day 24, the mice were sacrificed and the tumors were taken out and weighed. The tumor inhibition rate (%) was calculated after the effect of the medicament based on a tumor inhibition rate of the control group being zero.
The values determined were presented as mean ± standard error (Mean ± SD). The experimental data for each group was analyzed with One-way ANOVA method in the SPSS 18.0 statistical software, and p < 0.05 means that the differences are statistically significant.

**Table 4.1 TTD's effects on the body weight of nude mice (Mean ± SD)**

| Group | Dosage (mg/kg/time) | No. of animals | | body weight (g) | |
|---|---|---|---|---|---|
| | | Initial | Final | Initial | Final |
| Control | -- | 3 | 3 | 21.6±0.21 | 21.5±0.92 |
| TTD50 | 50 | 3 | 3 | 21.1±0.25 | 20.6±1.74 |

**Table 4.2 TTD's effects on the A549 transplanted tumor in nude mice (Mean ± SD)**

| Group | Dosage (mg/kg/time) | No. of animals | | Mass of tumor (g) | tumor inhibition rate (%) |
|---|---|---|---|---|---|
| | | Initial | Final | | |
| Control | -- | 3 | 3 | 0.37±0.23 | -- |
| TTD50 | 50 | 3 | 3 | 0.23±0.35 | 38.02 |

**Table 5.1 TTD's effects on the Hep G2 transplanted tumor in nude mice (Mean ± SD)**

| Group | Dosage (mg/kg/time) | No. of animals | | Mass of tumor (g) | tumor inhibition rate (%) |
|---|---|---|---|---|---|
| | | Initial | Final | | |
| Control | -- | 3 | 3 | 0.39±0.07 | -- |
| TTD50 | 50 | 3 | 3 | 0.53±0.32 | -33.36 |

### Analysis and discussion on Example 4:

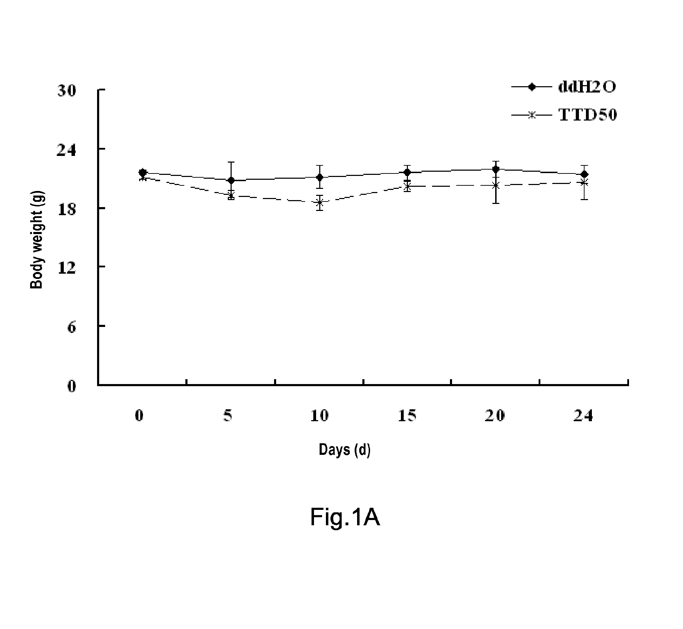

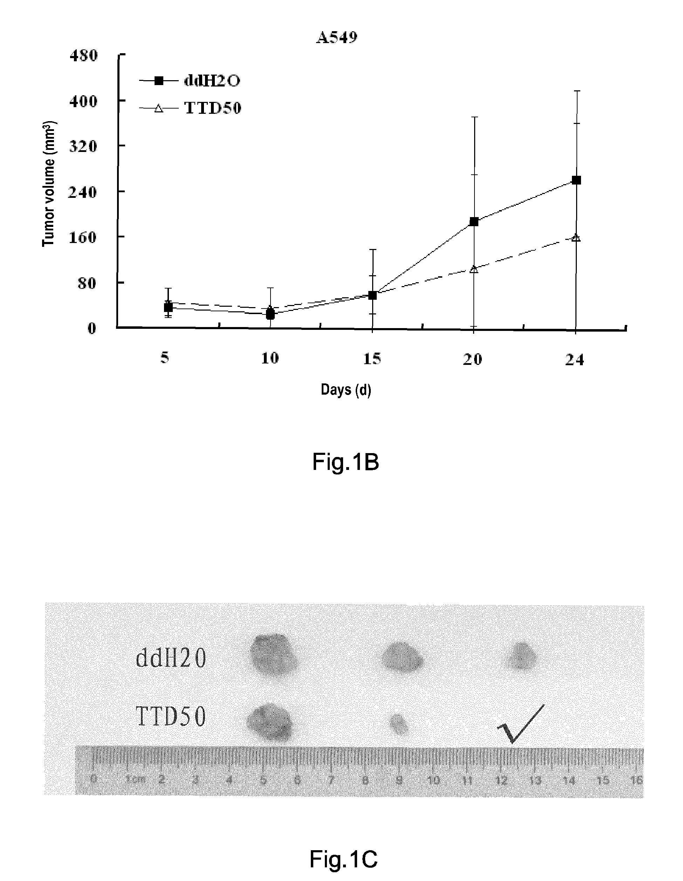

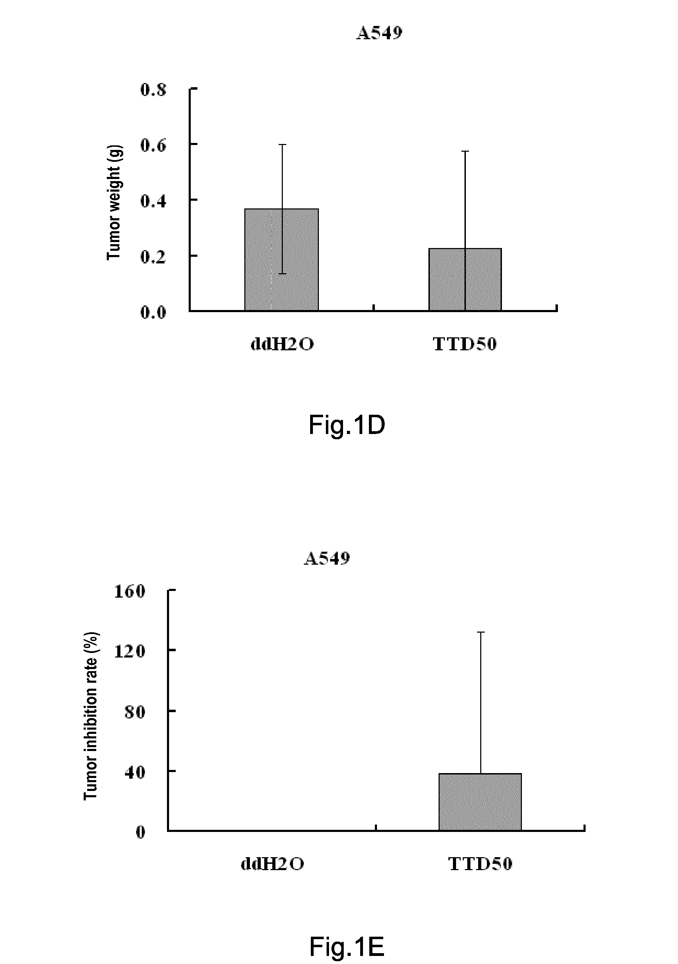

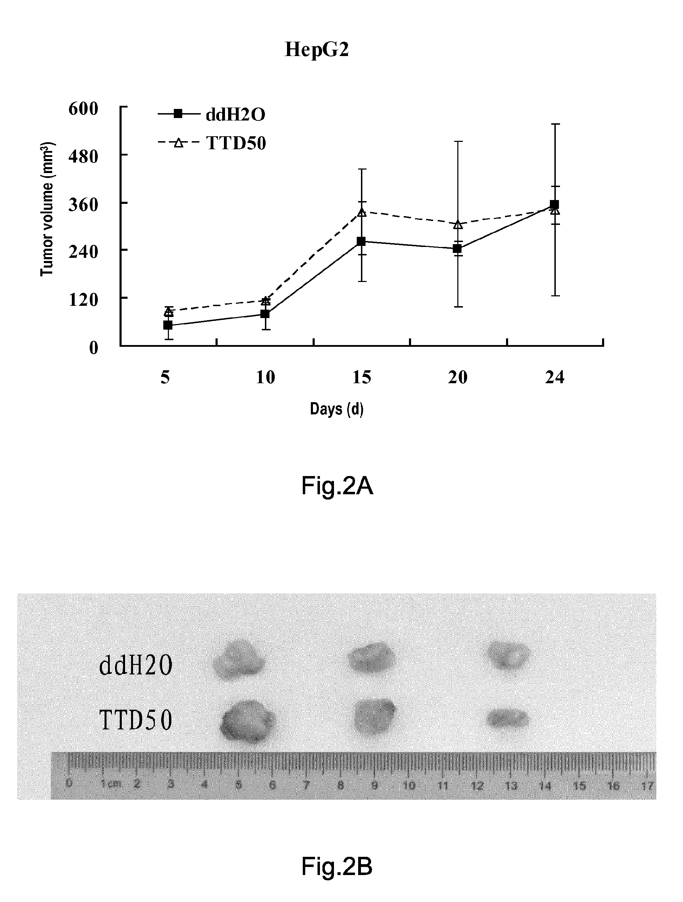

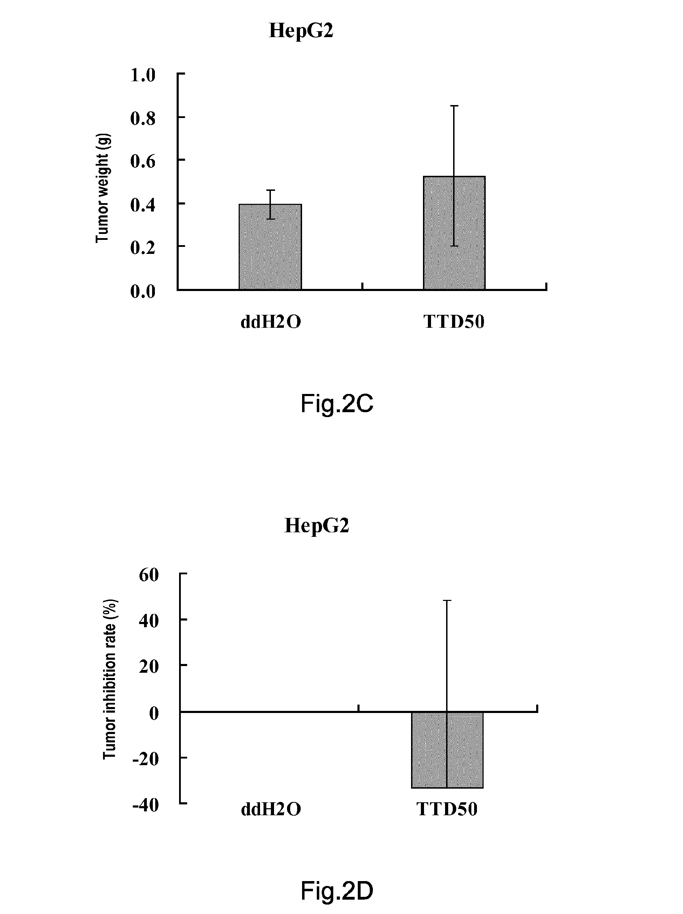

This experiment tested the effects of TTD on lung cancer and liver cancer transplanted tumor in nude mice, wherein TTD achieveed a tumor inhibition rate up to 38% on lung cancer at the dosage of this experiment and exhibited inhibitory effect (see Figures 1B to 1E). However, TTD did not exhibit good inhibitory effect on liver cancer in this experiment (see Figures 2A to 2D). It was deduced from the dynamic variation of the body weight of the animal that TTD causeed partial reduction in the body weight of the nude mice during the administration of this experiment. However, the body weight recovered gradually after the administration was terminated (see Figure 1A).

### Example 5: Acla A for the inhibition of lung cancer transplanted tumor in nude mice

(1) Experimental materials
Lung cancer cell lines: A549 (human non-small cell lung cancer cell line);
Animals: BALB/c-nu nude mice (immunodeficient mice), 8 weeks, female, purchased from Shanghai Laboratory Animal Center of Chinese Academy of Sciences, China.

(2) Reagents:
Jef (Jefitinib, LC laboratories)
Acla A (aclarubicin A, Shenzhen Main Luck Pharmaceuticals Inc.)

(3) Main apparatuses:
Cell incubator (Thermo Scientific 3111)
Laminar flow rack (Suhang Brand clean animal breeding cabinet, Type DJ-2).

(4) Experimental method
Under sterile conditions, the above tumor cells were collected in the logarithmic growth phase. A549 was injected by subcutaneous injection in an amount of 6×10⁶/0.2mL/nude mice (cell viability>90%) into the right subaxillary of the nude mice, thus establishing transplated tumor models of lung cancer in nude mice. The mice were administered from the second day after the inoculation. The experimental group was intragastrically administered at the experimentally designed amount, the negative control group was intragastrically administered with sterile water, and the positive control group is intragastrically administered with Jef. Each mouse was intragastrically administered in 0.4 ml each time and 2 times a day, at 8:00, and 14:00. The administrations were successive for 14 days. The day before administration was deemed as Day 0 and the body weight and tumor size of the mice were determined every 5 days to produce a dynamic plot on body weight and tumor growth. On Day 20, the mice were sacrificed and the tumors were taken out and weighed. The tumor inhibition rate (%) was calculated after the effect of the medicament based on a tumor inhibition rate of the control group being zero.
The values determined were presented as mean ± standard error (Mean ± SD). The experimental data for each group was analyzed with One-way ANOVA method in the SPSS 18.0 statistical software, and p < 0.05 means that the differences are statistically significant.

**Table 6.1 Acla A's effects on the A549 transplanted tumor in nude mice (Mean ± SD)**

| Group | Dosage (mg/kg/time) | No. of animals | | Body weight (g) | | Mass of tumor (g) | tumor inhibition rate (%) |
|---|---|---|---|---|---|---|---|
| | | Initial | Final | Initial | Final | | |
| Control | -- | 3 | 3 | 21.2±0.40 | 18.5±1.13 | 0.33±0.21 | -- |
| Jef50 | 50 | 3 | 3 | 21.1±0.46 | 17.3±2.54 | 0.38±0.37 | -14.34 |
| Acla A | 1 | 3 | 3 | 21.0±0.23 | 18.5±1.10 | 0.50±0.19 | -51.52 |

### Analysis and discussion on Example 5:

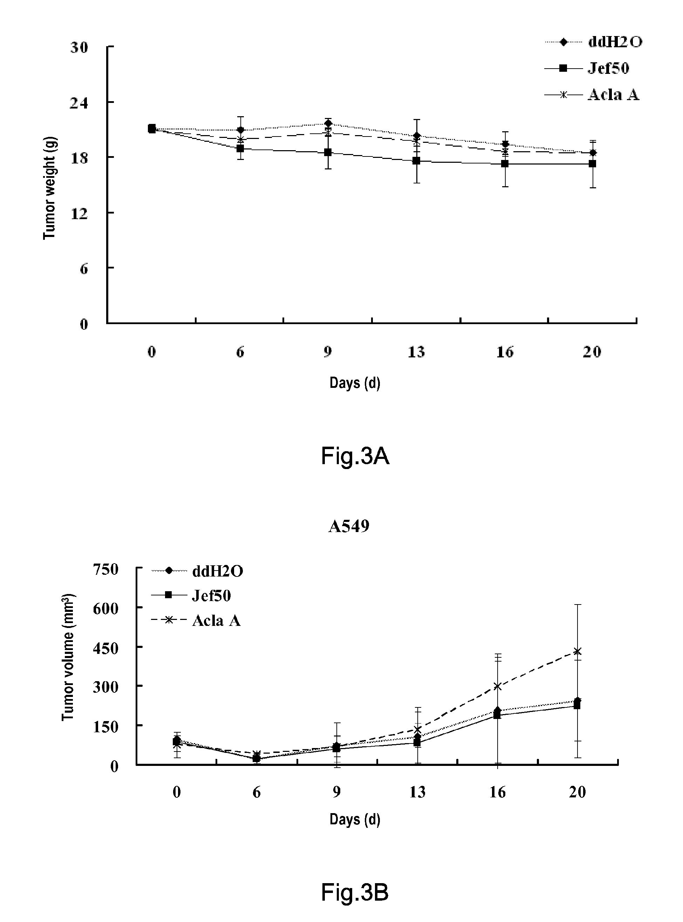

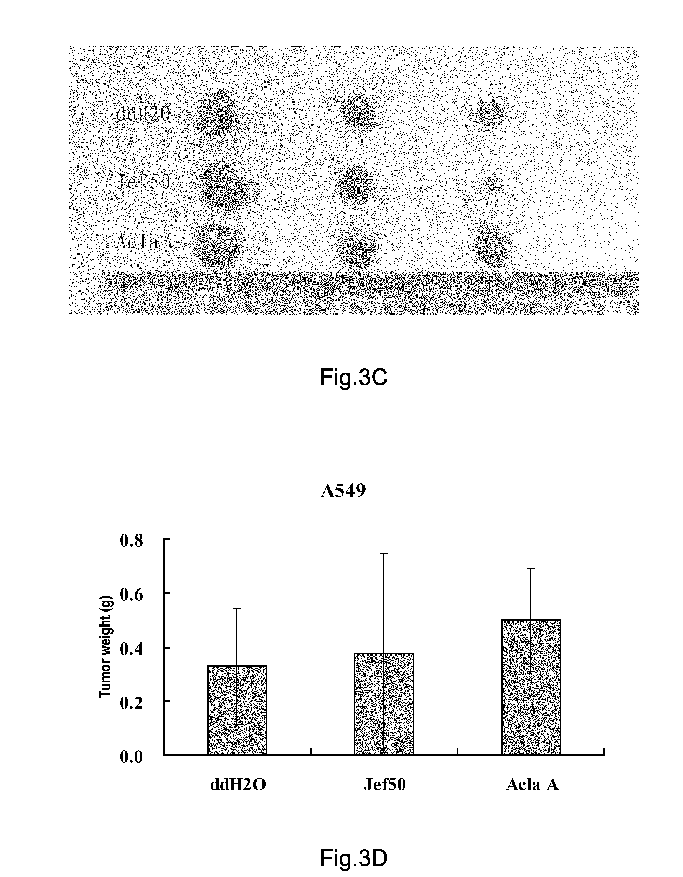

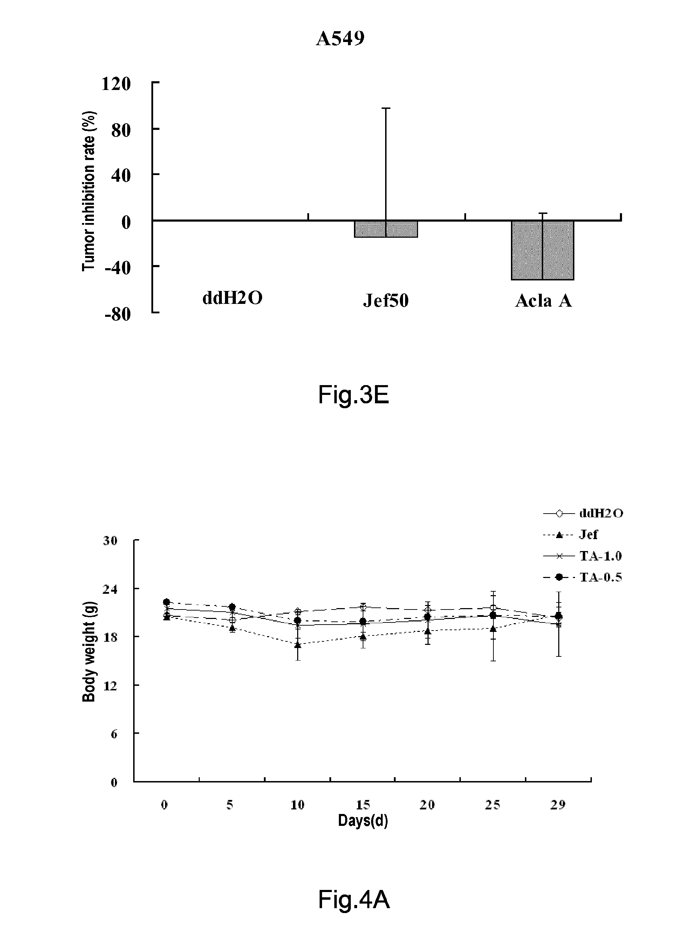

This experiment tested the effects of Acla A on lung cancer transplanted tumor in nude mice, wherein Acla A did not exhibit good inhibitory effect at the dosage used in this experiment (see Figures 3B to 3E). It was deduced from the dynamic variation of the body weight of the animal that Acla A caused partial reduction in the body weight of the nude mice during the administration of this experiment, which, however, recovered gradually after the administration is terminated (see Figure 3A).

### Example 6: Pharmaceutical combination of TTD and Acla A for the inhibition of lung cancer and liver cancer transplanted tumors in nude mice

(1) Experimental materials
Lung cancer cell lines: A549 (human non-small cell lung cancer cell line);
Liver cancer cell lines: Hep G2 (human hepatoma carcinoma cell);
Animals: BALB/c-nu nude mice (immunodeficient mice), 8 weeks, female, purchased from Shanghai Laboratory Animal Center of Chinese Academy of Sciences, China.

(2) Reagents:
TTD (tetrandrine, Jiangxi Jinfurong Pharmaceutical Co., Ltd.);
Jef (Jefitinib, LC laboratories);
Acla A (aclarubicin A, Shenzhen Main Luck Pharmaceuticals Inc.);
TA (the combination of tetrandrine and aclarubicin A)

(3) Main apparatuses:
Cell incubator (Thermo Scientific 3111)
Laminar flow rack (Suhang Brand clean animal breeding cabinet, Type DJ-2).

(4) Experimental method
Under sterile conditions, the above tumor cells were collected in the logarithmic growth phase. A549 was injected by subcutaneous injection in an amount of 1×10⁷/0.2 mL/nude mice (cell viability>90%) into the right subaxillary of the nude mice, whereas HepG2 was injected by subcutaneous injection in an amount of 1×10⁷/0.2 mL/nude mice (cell viability>90%) into the left subaxillary of the nude mice, thus establishing transplated tumor models of lung cancer and liver cancer respectively in nude mice. The mice were administered from the second day after the inoculation. The experimental group was intragastrically administered at the experimentally designed amount, the negative control group was intragastrically administered with sterile water, and the positive control group was intragastrically administered with Jef. Each mouse was intragastrically administered in 0.4 ml each time and 3 times a day, at 8:00, 14:00 and 20:00. The administrations were successive for 10 days. The day before administration was deemed as Day 0 and the body weight and tumor size of the mice were determined every 5 days to produce a dynamic plot on body weight and tumor growth. On Day 29, the mice were sacrificed and the tumors were taken out and weighed. The tumor inhibition rate (%) was calculated after the effect of the medicament based on a tumor inhibition rate of the control group being zero.
The values determined were presented as mean ± standard error (Mean ± SD). The experimental data for each group was analyzed with One-way ANOVA method in the SPSS 18.0 statistical software, and p < 0.05 means that the differences are statistically significant.

**Table 7.1 TTD and Acla A's combined effects on the A549 transplanted tumor of nude mice (Mean ± SD)**

| Group | Dosage (mg/kg/time) | No. of animals | | Body weight (g) | | Mass of tumor(g) | tumor inhibition rate (%) |
|---|---|---|---|---|---|---|---|
| | | Initial | Final | Initial | Final | | |
| Control | -- | 3 | 3 | 20.7±0.25 | 20.3±0.68 | 1.13±0.21 | -- |
| Jef | 50 | 3 | 2 | 20.5±0.10 | 20.8±1.48 | 1.52±1.00 | -34.11 |
| TA-1.0 | TTD50mg+ Acla A 1.0 mg | 3 | 3 | 21.5±0.70 | 19.5±4.01 | 0.13±0.20 | 88.93 |
| TA-0.5 | TTD50mg+Acla A 0.5mg | 3 | 3 | 22.3±0.31 | 20.6±1.17 | 0.86±0.74 | 23.55 |

**Table 8.1 TTD and Acla A's combined effects on the Hep G2 transplanted tumor of nude mice (Mean ± SD)**

| Group | Dosage (mg/kg/time) | No. of animals | | Body weight (g) | | Mass of tumor (g) | tumor inhibition rate (%) |
|---|---|---|---|---|---|---|---|
| | | Initial | Final | Initial | Final | | |
| Control | -- | 3 | 3 | 20.7±0.25 | 20.3±0.68 | 0.68±0.22 | **--** |
| Jef | 50 | 3 | 2 | 20.5±0.10 | 20.8±1.48 | 0.31±0.10 | 54.08* |
| TA-1.0 | TTD50+ Acla A1.0 | 3 | 3 | 21.5±0.70 | 19.5±4.01 | 0.32±0.14* | 53.56* |
| TA-0.5 | TTD50+ Acla A0.5 | 3 | 3 | 22.3±0.31 | 20.6±1.17 | 0.34±0.21 | 49.80* |

| | | | | | | | |
|---|---|---|---|---|---|---|---|
| Note: * denotes P<0.05 as compared with the control groups | | | | | | | |

### Analysis and discussion on Example 6:

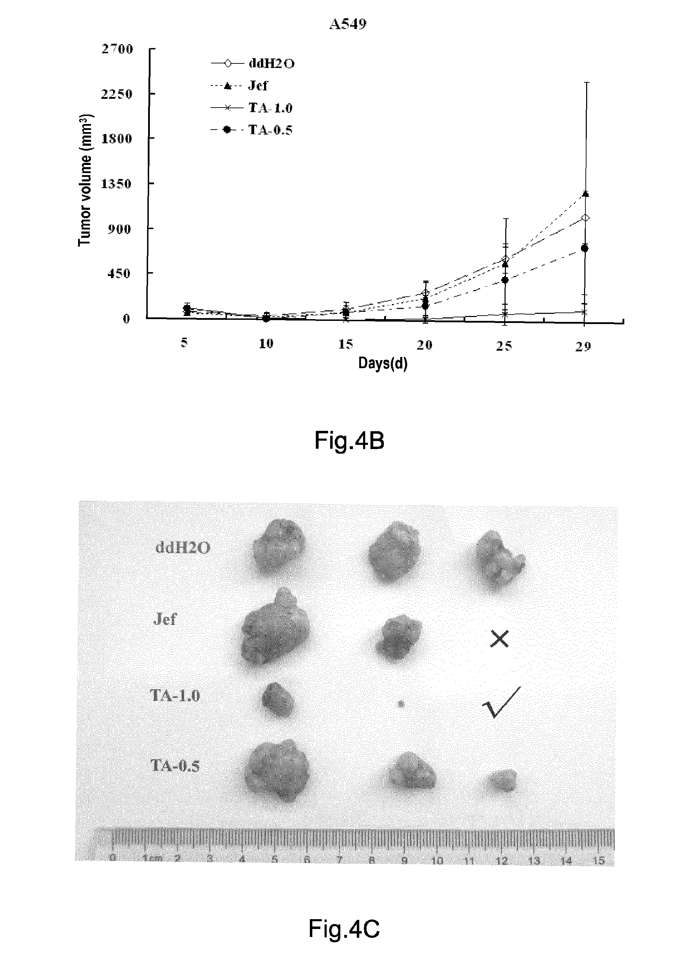

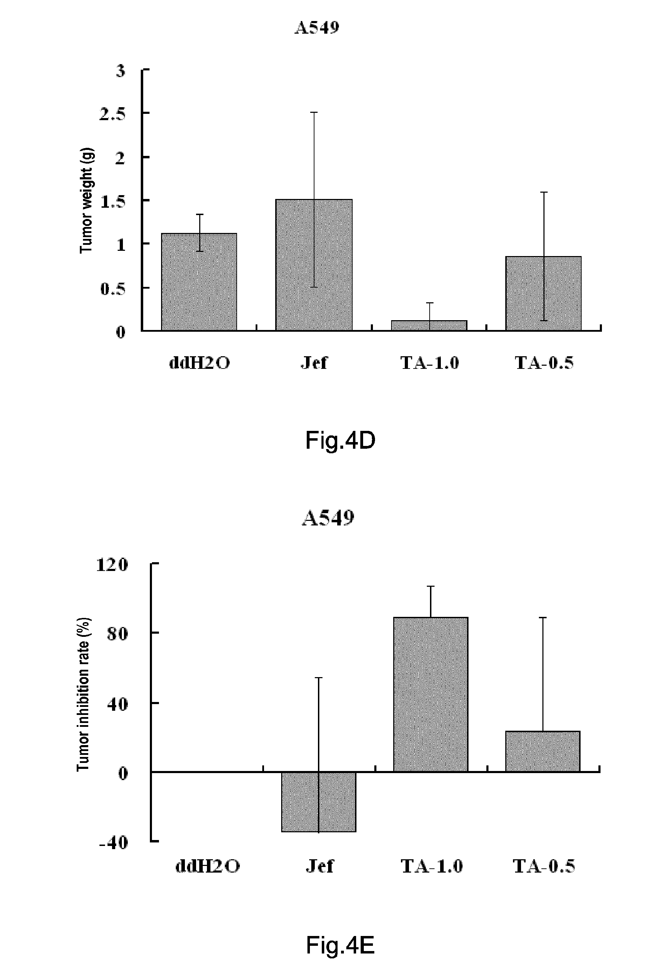

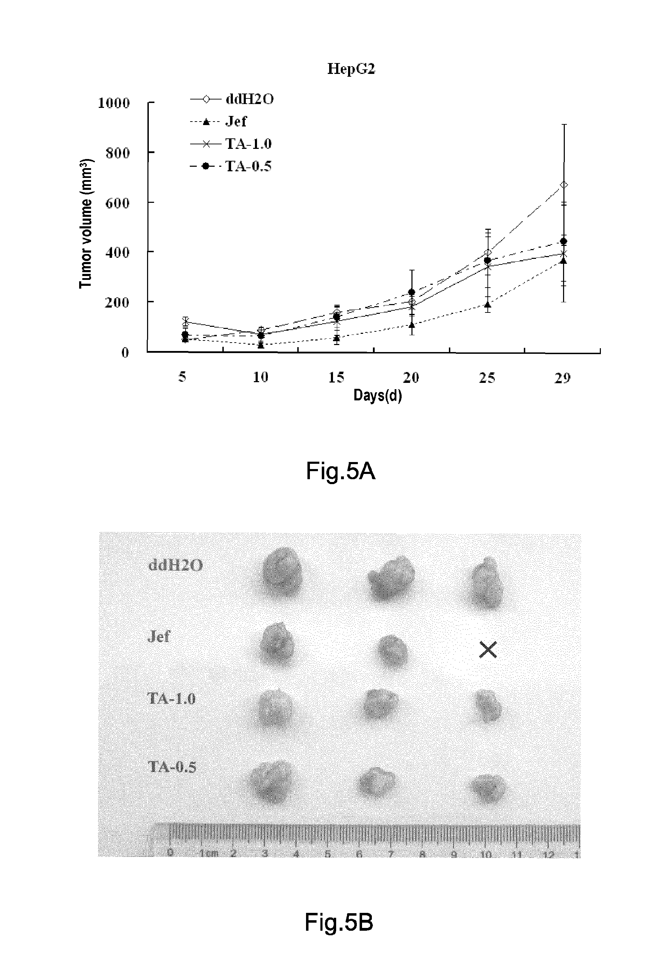

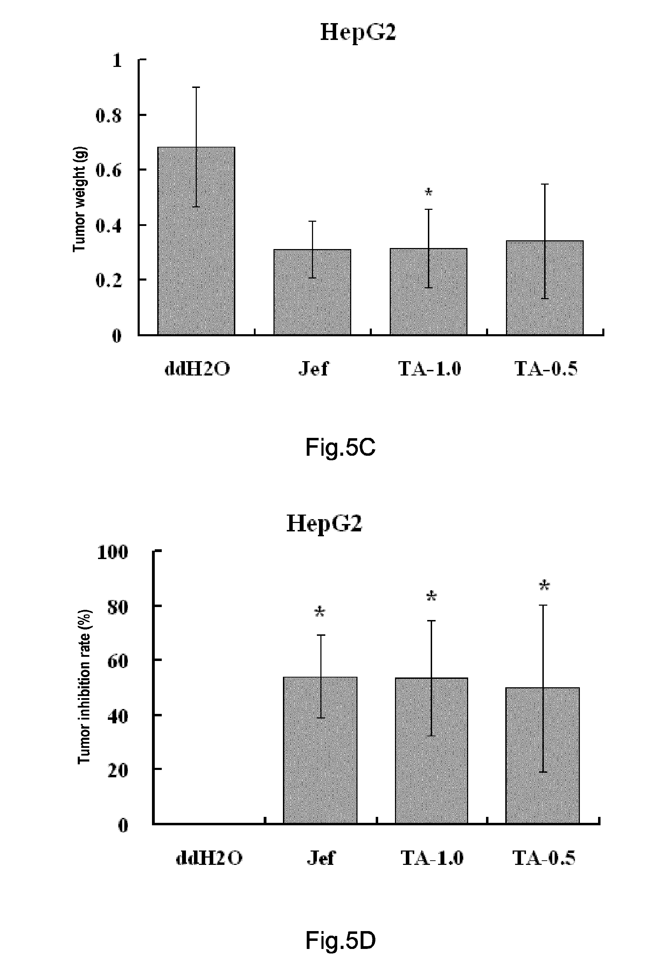

This experiment tested the combined effects of TTD and Acla A on lung cancer and liver cancer transplanted tumor in nude mice. As is shown in Figures 4B to 4E and Figures 5A to 5D, the combined dose of TTD and Acla A at 1.0mg/kg./time achieved a tumor inhibition rate up to 89% on lung cancer and achieved a tumor inhibition rate up to 53% on liver cancer. Meanwhile, the combined dose of TTD and Acla A at 0.5mg/kg./time achieved a tumor inhibition rate up to 24% on lung cancer and achieved a tumor inhibition rate up to 50% on liver cancer. The use of these two drugs in combination achieved significant difference on the inhibition rate of liver cancer as compared with the control groups. It was deduced from the dynamic variation of the body weight of the animal that the use of these two drugs in combination did not cause significant reduction in the body weight of the nude mice in this experiment, whereas animals of the positive control group Jef showed significant reduction in the body weight (see Figure 4A).

## Claims

1. An antitumor pharmaceutical combination, comprising a bisbenzylisoquinoline alkaloid of Formula (I) or a pharmaceutically acceptable salt thereof and an anthracene compound of Formula (II) or a pharmaceutically acceptable salt thereof as active components, 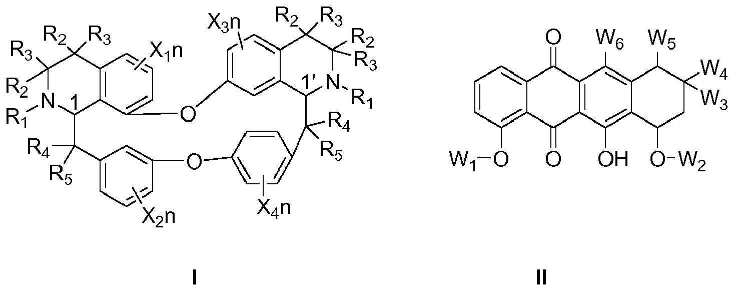 in Formula (I),
R₁ is H or linear or branched alkyl comprising 1 to 10 carbon atoms;
R₂ and R₃ each independently is H, substituted acyl, linear or branched alkyl, wherein the alkyl can be interrupted by O, N, or S heteroatoms; or R₂ and R₃ together represents O or S;
R₄ and R₅ each independently is H, substituted acyl, linear or branched alkyl, wherein the alkyl can be interrupted by O, N, or S heteroatoms; or R₄ and R₅ together represents O or S;
X₁, X₂, X₃, and X₄ can be identical or different, each of which independently is halogen atom, hydroxyl, or linear or branched alkyl comprising 1 to 10 carbon atoms, or alkoxy, or acyloxy, and n is an integer of from 0 to 4;
C(1) and C(1') are of a stereoisomer configuration selected from RR, SS, 1S1'R, 1R1'S;
in Formula (II),
W₁ is H, methyl, substituted or unsubstituted C₁-C₆ alkyl;
W₂ is H, glycosyl, or amino-substituted glycosyl;
W₃ and W₄ each is H, hydroxyl, substituted or unsubstituted alkyl, or substituted carbonyl;
W₅ is H, substituted carbonyl or ester group;
W₆ is H, or hydroxyl;
wherein the aforementioned substituent is selected from the group consisting of halogen, amino, C₁-C₆ substituted amino, nitro, cyano, hydroxyl, C₁-C₆ alkoxy, mercapto, and C₁-C₆ alkylthio; wherein the substituent(s) can be one or more for each occurrence, identical or different, each substituting an H atom.

2. The antitumor pharmaceutical combination according to claim 1, wherein the bisbenzylisoquinoline alkaloid of Formula (I) is tetrandrine or a pharmaceutically acceptable salt thereof.

3. The antitumor pharmaceutical combination according to claim 1, wherein the anthracene compound of Formula (II) is Aclarubicin A.

4. The antitumor pharmaceutical combination according to any one of claims 1 to 3, **characterized in that** the bisbenzylisoquinoline alkaloid of Formula (I) comprises 0.1-99.9% of the total weight of the active components of the combination; and the anthracene compound of Formula (II) comprises 0.1-99.9% of the total weight of the active components of the combination.

5. The antitumor pharmaceutical combination according to any one of claims 1 to 3, **characterized in that** the bisbenzylisoquinoline alkaloid of Formula (I) comprises 1-99% of the total weight of the active components of the combination; and the anthracene compound of Formula (II) comprises 1-99% of the total weight of the active components of the combination.

6. The antitumor pharmaceutical combination according to any one of claims 1 to 3, **characterized in that** the bisbenzylisoquinoline alkaloid of Formula (I) comprises 10-90% of the total weight of the active components of the combination; and the anthracene compound of Formula (II) comprises 10-90% of the total weight of the active components of the combination.

7. The antitumor pharmaceutical combination according to any one of claims 1 to 3, **characterized in that** the bisbenzylisoquinoline alkaloid of Formula (I) comprises 20-80% of the total weight of the active components of the combination; and the anthracene compound of Formula (II) comprises 20-80% of the total weight of the active components of the combination.

8. Use of the bisbenzylisoquinoline alkaloid of Formula (I) or a pharmaceutically acceptable salt thereof and the anthracene compound of Formula (II) or a pharmaceutically acceptable salt thereof as recited in any of claims 1-7 as the active components in manufacturing an antitumor medicament.

9. A method for treating a subject suffering from tumor, comprising administrating to the subject in need thereof an effective amount of the bisbenzylisoquinoline alkaloid of Formula (I) or a pharmaceutically acceptable salt thereof and the anthracene compound of Formula (II) or a pharmaceutically acceptable salt thereof as recited in any of claims 1-7.

10. The pharmaceutical combination according to any of claims 1-7 for use as an antitumor agent.

11. The use, method or pharmaceutical combination respectively according to claims 8, 9 or 10, wherein the tumor is selected from leukemia, multiple myeloma, lymphoma, liver cancer, gastric cancer, breast cancer, cholangiocellular carcinoma, pancreatic cancer, lung cancer, colorectal carcinoma, osteosarcoma, human cervical cancer, glioma, nasopharyngeal carcinoma, laryngeal carcinoma, esophageal cancer, middle ear tumor, melanoma, ovarian carcinoma, renal carcinoma, metrocarcinoma, osteosarcoma, and prostate cancer.
